# EUROPEAN PATENT SPECIFICATION

(11) **EP 2 344 121 B1**
(45) Date of publication and mention of the grant of the patent: **12.07.2017**
(21) Application number: 09744012.7
(22) Date of filing: 22.10.2009
(51) Int. Cl.: A61K 47/36, A61K 9/16, A61P 15/02

(54) **VAGINAL PELLETS COMPRISING DEBRANCHED STARCH**
VAGINALPELLETS MIT ENTZWEIGTER STÄRKE
PELLETS VAGINAUX COMPRENANT UN AMIDON DERAMIFIE

(30) Priority: 23.10.2008 US 107784 P
(43) Date of publication of application: 20.07.2011
(73) Proprietor: Henkel IP & Holding GmbH, 40589 Düsseldorf (DE)
(72) Inventor: REMON, Jean, Paul, B-9090 Melle (BE); VERVAET, Chris, B-8870 Izegem (BE); FOREMAN, Paul, Somerville NJ 08876 (US)
(74) Representative: Henkel IP Department
(86) International application number: PCT/US2009/061662
(87) International publication number: WO 2010/048389

(56) References cited:
- WO-A1-99/09066
- WO-A1-99/53902
- US-A- 5 468 286
- US-A1- 2006 246 192
- MULHBACHER J ET AL: "Mucoadhesive properties of cross-linked high amylose starch derivatives" INTERNATIONAL JOURNAL OF BIOLOGICAL MACROMOLECULES, ELSEVIER BV, NL LNKD- DOI:10.1016/J.IJBIOMAC.2006.05.003, vol. 40, no. 1, 15 December 2006 (2006-12-15), pages 9-14, XP025096147 ISSN: 0141-8130 [retrieved on 2006-12-15]
- POELVOORDE N ET AL: "In vivo evaluation of the vaginal distribution and retention of a multi-particulate pellet formulation" EUROPEAN JOURNAL OF PHARMACEUTICS AND BIOPHARMACEUTICS, ELSEVIER SCIENCE PUBLISHERS B.V., AMSTERDAM, NL LNKD- DOI:10.1016/J.EJPB.2009.06.005, vol. 73, no. 2, 1 October 2009 (2009-10-01), pages 280-284, XP026652689 ISSN: 0939-6411 [retrieved on 2009-06-12]
- SANTIAGO G L D S ET AL: "A pilot study evaluating the safety of vaginal administration of a multi-particulate pellet formulation" EUROPEAN JOURNAL OF PHARMACEUTICS AND BIOPHARMACEUTICS, ELSEVIER SCIENCE PUBLISHERS B.V., AMSTERDAM, NL LNKD- DOI:10.1016/J.EJPB.2009.08.009, vol. 73, no. 3, 1 November 2009 (2009-11-01), pages 399-403, XP026736266 ISSN: 0939-6411 [retrieved on 2009-09-04]
- DUKIC ET AL: "Development of starch-based pellets via extrusion/spheronisation" EUROPEAN JOURNAL OF PHARMACEUTICS AND BIOPHARMACEUTICS, ELSEVIER SCIENCE PUBLISHERS B.V., AMSTERDAM, NL LNKD- DOI:10.1016/J.EJPB.2006.08.015, vol. 66, no. 1, 16 March 2007 (2007-03-16) , pages 83-94, XP005938640 ISSN: 0939-6411

## Description

### FIELD OF THE INVENTION

The invention relates to a composition comprising pellets for use in the treatment of a disease by vaginal administration, said pellets comprising a debranched starch, which has been enzymatically hydrolyzed by at least one enzyme capable of cleaving the 1,6-linkages of the starch molecule, and a therapeutic agent.

### BACKGROUND OF THE INVENTION

Vaginal drug delivery is a promising route for local and systemic drug delivery. By this way of delivery the hepatic first-pass effect can be avoided and due to the presence of a dense network of blood vessels surrounding the vagina rapid absorption can be obtained (N.J. Alexander, E. Baker, M. Kaptein, U. Karck, L. Miller, E. Zampaglione, Fertility and Sterility 82(1) (2004) 1-12; A. Hussain, F. Ahsan, J. Control. Release 103(2) (2005) 301-313). Moreover, the vaginal mucosa has a high permeability for large molecular weight drugs, like peptides and proteins (J.L. Richardson, L. Illum, Penetration enhancement for polypeptides through epithelia. D. Routes of delivery - case-studies .8. The vaginal route of peptide and protein drug delivery. Adv. Drug Deliv. Rev. 8(2-3) (1992) 341-366). Despite the good characteristics of the vagina for drug therapy, the development of a good vaginal delivery system remains an issue.

A good vaginal formulation should have a sufficiently long retention time to maximize drug release. Furthermore, a proper spreading of the formulation over the vaginal epithelium is important to obtain a fast absorption, but also to provide a good effect in case of local treatment. Concerning patient compliance, the formulation should be easy to administer and should not cause discomfort feelings within the user population.

Different types of formulations are on the market, each with their advantages and disadvantages. The main group of vaginal delivery forms on the market are semi-solid dosage forms like creams and gels containing hormones, antibiotics, fungicides etc. Gels and creams have shown to be good delivery systems with ease in formulation and administration and a fast release of drugs. However, these vaginal forms are messy to apply, can leak in the undergarments and give an uncomfortable feeling to the user population (A.C. Broumas, L.A. Basara, Advances in Therapy 17(3) (2000) 159-166; M.E. Bentley, K.M. Morrow, A. Fullem, M.A. Chesney, S.D. Horton, Z. Rosenberg, K.H. Mayer, Family Planning Perspectives 32(4) (2000) 184-188; M. Justin-Temu, F. Damian, R. Kinget, G. Van den Mooter, Journal of Women's Health 13(7) (2004) 834-844). Moreover, semi-solid formulation may not provide an exact dose due to a nonuniform distribution and leakage. Multiple administrations can be necessary as there is no long retention time (A. Hussain, F. Ahsan, J. Control. Release 103(2) (2005) 301-313). Great differences in retention of semi-solid formulations between individuals were reported, varying from 1 to 81 % retained of the initial dose after 24 h (B.E. Chatterton, S. Penglis, J.C. Kovacs, B. Presnell, B. Hunt, Int. J. Pharm. 271(1-2) (2004) 137-143). Brown et al. reported a loss varying from 97 to 9% two hours post-dosing in post-menopausal women (J. Brown, G. Hooper, C.J. Kenyon, S. Haines, J. Burt, J.M. Humphries, S.P. Newman, S.S. Davis, R.A. Sparrow, I.R. Wilding, Pharm. Res. 14(8) (1997) 1073-1078). Bamhardt et al. observed very little vaginal mucosal coverage 24h after application of a microbicide C31G vaginal gel (K.T. Barnhart, E.S. Pretorius, K. Timbers, D. Shera, M. Shabbout, D. Malamud, Contraception 71(5) (2005) 357-361).

A second group are the tablet formulations, the most common dosage form used for oral drug delivery. Tablets are easy to manufacture, easy inserted and have a low production cost. Vaginal disintegration of conventional tablets, however, can take too much time and due to gravity the tablets are rapidly cleared from the vagina. This can be circumvented by the use of bioadhesive vaginal tablets, but in some studies loss of those bioadhesive tablets was reported (J. Voorspoels, M. Casteels, J.P. Remon, M. Temmerman, European Journal of Obstetrics, Gynecology and Reproductive Biology 105(1) (2002) 64-66). Also, suppositories gave a messy feeling, too fast release and moreover this dosage form can not be stored at high temperatures. Suppositories are mostly used for cervical ripening prior to childbirth.

Vaginal rings are torus-shaped polymeric devices, most often silicone based, designed to release one or more incorporated drugs in a controlled fashion (N.J. Alexander, E. Baker, M. Kaptein, U. Karck, L. Miller, E. Zampaglione, Fertility and Sterility 82(1) (2004) 1-12). Compared to other vaginal delivery systems, they offer certain advantages, including accurate and sustained dosing. Vaginal rings deliver their drugs very locally in the vagina, while moreover, not all drugs can be incorporated into the device and their production is expensive.

There is a need for the development of a new vaginal delivery form which assures an acceptable retention time, combined with a good spreading over the vaginal epithelium. The current invention addresses this need.

### SUMMARY OF THE INVENTION

The invention provides the art with excipients useful in the manufacture of pellets, methods of making pellets, and use of pellets in the treatment or amelioration of various conditions and disease states using a vaginal route of administration.

The present invention relates toa composition comprising pellets for use in the treatment of a disease by vaginal administration, said pellets comprising a debranched starch, which has been enzymatically hydrolyzed by at least one enzyme capable of cleaving the 1,6-linkages of the starch molecule, and a therapeutic agent. One method of manufacturing the pellets is via extrusion/spheronization.

It has been discovered that pharmaceutical pellets made using a crystalline debranched amylase resistant starch are particularly useful for the vaginal delivery of active agents. The invention provides compositions and methods for treating or preventing of various conditions. Conditions to be treated include, but are not limited to vaginosis and vaginitis.

The composition according to the present invention may be in the form of a pessaries, tablets or capsules which comprise specific pellets and an active ingredient. Preferably, the pellets comprise a crystalline debranched amylase resistant starch and an active ingredient. Spherical pellets prepared using extrusion/spheronization and having an average diameter of 300 - 1000 µm comprise one preferred embodiment. Many drugs can be incorporated in these spheres. Vaginal drug delivery using the pellets of the invention combines the advantage of the fast spreading semi-solid formulations with a better retention time.

### DETAILED DESCRIPTION OF THE INVENTION

Pellets have been used in the art as a carrier for pharmacologically active ingredients. Methods of manufacturing pellets for pharmaceutical use in both conventional (immediate release) and extended release single dosage forms include extrusion/spheronization. Due to its rheological properties, microcrystalline cellulose (MCC) has typically been used as the main excipient in the production of pellets via extrusion/spheronization. MCC-based pellets, however, do not disintegrate and drug release occurs by way of diffusion through an insoluble inert matrix. Due to this limitation, alternatives to MCC formulations have been investigated.

A specific starch grade has been identified as a material suitable for use as an excipient for pellets prepared via extrusion/spheronization. U.S. patent application publication No. 2006/0246192. This starch is a modified starch obtained by an enzymatic debranching of amylose-rich starch. After debranching, the starch is retrograded and further isolated by extrusion or drying. U.S. Patent No. 5,281,276. This process yields a crystalline, high-amylose material consisting of D-anhydroglucose units (linked by α-1,4-D-glycosidic bonds) organized into double-helical crystalline chains. It is referred to as a resistant starch because the α-1,4-D-glycosidic bonds between the D-anhydroglucose monomers are inaccessible to α-amylase in the small intestine due to the formation of a double-helical crystalline chain structure.

It has now been discovered that disintegrating starch-based pellets provide the art with formulations that can advantageously be administered by vaginal administration. The formulations combine the advantage of the fast spreading semi-solid formulations with a better retention time compared to tablets and semi-solids because of the small size (preferably 300 - 1000 µm) which supersede gravitational effects.

The invention provides the art with pharmaceutical compositions and methods of preparing pharmaceutical compositions are disclosed. The compositions of the present invention can be used for the treatment of vaginal conditions, including vaginal infections, and to methods of treating vaginal conditions by the administration of said composition. It has been discovered that particular types of starch, in particular starch pellets made from crystalline debranched amylase resistant starch, exhibit good dispersion characteristics following vaginal application.

Crystalline debranched resistant starches may be prepared as described in U.S. Patent No. 5,281,276, the disclosure of which is incorporated herein by reference. Typically, such starches are modified by enzymatic debranching of an amylose-rich starch. After debranching, the starch is retrograded and further isolated by extrusion or drying. This process yields a crystalline, high-amylose material consisting of D-anhydroglucose units (linked by α-1,4-D-glycosidic bonds) organized into double-helical crystalline chains. It is referred to as a resistant starch because the α-1,4-D-glycosidic bonds between the D-anhydroglucose monomers are inaccessible to α-amylase in the small intestine due to the formation of a double-helical crystalline chain structure. Such starches are commercially available from Henkel Corporation, Bridgewater, NJ under the tradename VELOX™ MCS (and formerly available under the tradename UNI-PURE^{®} EX starch from National Starch and Chemical Company).

In one preferred embodiment, pellets are prepared via extrusion/spheronization. Pellets of the type described for use in U.S. patent application publication No. 2006/0246192 may be used in the present invention.

Pellets prepared from starch have been discovered to be particularly advantageous when used for the delivery of active agents, including pharmacologically active drugs by a vaginal mode of administration The composition may be in the form of pessaries, tablets or capsules which comprise the specific pellets of the present invention and an active ingredient. Alternatively, pellets may be administered directly, without encapsulation, be means of a suitable delivery device. The pellets comprise a crystalline debranched resistant starch and an active ingredient as described in the claims. Pellets of the invention may be advantageously prepared using extrusion/spheronization. It is to be understood that the pellets used in the practice of the invention are not limited to pellets prepared by extrusion/spheronization, but include other techniques such as for example rotary fluid bed and shear granulation.

The pellets may be used for both immediate- and controlled-release dosage forms and offer greater flexibility in dosing and drug release design. Being a multiparticulate solid dosage form, two or more active agents can be combined in any ratio in a single dosage form. As such, combination products can be prepared that contain active agents that are incompatible or have varying release profiles. The vaginal use compositions of the invention may contain a single active, but may contain a combination of two or more actives.

The term patient is used herein to include female mammals, both human and non-human, including companion animals such as dogs, cats and horses and livestock such as cattle and swine. Human patients include adults, children and infants.

The term "drug" is to be construed herein in its broadest sense to mean any agent which is intended to produce some therapeutic benefit. The agent may or may not be pharmacologically active, but will be "bioactive" in the sense that it has an, at least perceived, effect on the human body. The agent may be used to treat or alter a condition, which may or may not be pathological, i.e., a disease state, condition. "Drug", "bioactive agent," "preparation," "medicament," "therapeutic agent," "physiological agent" and "pharmaceutical agent" are used interchangeably herein and include substances for use in the diagnosis, cure, mitigation, arrest, treatment or prevention of a condition or disease state or to affect the structure or function of the body. Agents that function to, e.g., soften and moisturize, are included in this term. The term "treatment" is used broadly to encompass prevention, alteration, cure and control of the condition.

The drug is present in the composition of the invention in a therapeutically effective amount, i.e., an amount effective to bring about a desired therapeutic result in the treatment of a condition to which the preparation of this invention is to be applied. An effective amount of a drug means a nontoxic but sufficient amount of a drug to provide the selected effect over a specific period of time. The amount that constitutes a therapeutically effective amount varies according to the particular drug or agent incorporated in the composition, the condition being treated, any drugs being co-administered with the selected drug, desired duration of treatment, and other such considerations. Such an amount is readily determinable by the skilled practitioner.

Drugs that can be included in the composition of the invention include substances capable of a local or a systemic effect. Treatment areas where the composition of the invention finds use include drugs or other agents for the treatment of bacterial and fungal infections, vaginal itchiness, vaginal dryness, and high vaginal acidity. It is to be understood that this list is not exhaustive and is provided merely as being illustrative of conditions that can be treated using the compositions and methods of the invention.

Bacterial vaginosis and related symptomology is generally caused by the substitution, in the vaginal region, of a large number of anaerobic bacteria for lactobacilli. Anaerobic bacteria causing symptoms of infection include Bacteroides, Peptostreptococcus, Peptococcus, and Mobiluncus G. vaginalis. Symptoms of vaginitis are typically due to a local infection due to Trichomonas vaginalis or Candida (in particular, Candida albicans). Symptoms include itch, secretion, dryness and may include dyspareunia. These conditions may be caused by, or be secondary to, diabetes, parathyroid insufficiency, altered defenses of the host organism, corticosteroid treatment, broad-spectrum antibiotic treatment, oral contraceptive drugs and pregnancy.

The compositions of the invention may also be used to treat inflammatory pelvic diseases, or to treat infections that occur after gynecological operations, childbirth, abortion and infections correlated with use of intrauterine devices for preventing pregnancy. Conditions such as low vaginal moisture or vaginal dryness, vaginal acidity and vaginal atrophy may also be treated using the compositions of the invention.

A variety of starch compatible active agents may be employed in this invention. The particular nature of the active ingredient is not critical, and pharmaceutical and non-pharmaceutical active ingredients may be used.

Examples of pharmaceutical products which can be administered in accordance with the invention include but are not limited to amino acids such as valine, leucine, and threonine, oligopeptides, polypeptides and anti-fungi drugs such as Fluconazole, Terconazole, Tioconazole, Butoconazole, Ketoconazole, Itraconazole, Econazole, Miconazole, and Cannitracin, analgesics, steroids, sexual hormones, vaginal probiotics, and other agents such as for example hyaluronic acid. It is to be understood that agents to be administered include the pharmaceutically acceptable salts thereof as well.

In the practice of the invention, crystalline debranched starch as disclosed in claim 1 is used as an excipient, and in one preferred embodiment as the main excipient, in the preparation of pharmaceutical pellets, which may desirably be manufactured via extrusion-spheronization. Such excipients are useful in any dry dosage form, including tablets and capsules, for either immediate or sustained release. The starch excipients can be used as a total replacement for microcrystalline cellulose or can be used as a partial replacement in combination with microcrystalline cellulose and/or other cellulose derivatives.

The term dosage form is intended in its broadest sense to mean not only pharmaceutical dosage forms which employ excipients to deliver active agent(s) and includes tablets (such as immediate release, sustained release, controlled release, modified release, and effervescent), capsules, pellets, and granules, but also non-pharmaceutical forms of these products.

Controlled release, as used herein, is intended to mean a method and composition for making an active ingredient available to the biological system of a host. Controlled release includes the use of instantaneous release, delayed release, and sustained release. "Instantaneous release" refers to immediate release to the biosystem of the host. "Delayed release" means the active ingredient is not made available to the host until some time delay after administration. "Sustained Release" generally refers to release of active ingredient whereby the level of active ingredient available to the host is maintained at some level over a period of time. The method of effecting each type of release can be varied. For example, the active ingredient can be associated physically and/or chemically with a surfactant, a chelating agent, etc. Alternatively, the active ingredient can be masked by a coating, a laminate, etc. Regardless of the method of providing the desired release pattern, the present invention contemplates delivery of a controlled release system that utilizes one or more of the "release" methods and compositions.

The term pellet is being used herein to refer to a substantially spherical solid particle whose diameter size may range from about 100 microns to about 3 mm.

Excipient is used herein to include, in addition to the modified starch component, binders, fillers, and all other ingredients which are pharmacologically inert. Typically a dosage form will optionally consist of several inert materials, referred to as excipients, in addition to the active ingredient, which is present in amounts sufficient to accomplish the desired pharmacological effect. These excipients are generally classified according to their functions, such as fillers (also called bulking agents and diluents), binders which hold the ingredients together, binder-fillers which perform both functions and disintegrants which help the dosage form to break apart and release the active ingredient when placed in a fluid environment. Use of a modified starch as the "main" excipient" means that the starch component is present in the greatest amount in relation to each of the other excipients used. Use of a modified starch as the primary excipient means the starch component comprises more than 50% of the total excipients by weight, exclusive of water or other processing solvent which is removed by drying. The term debranched starch is used herein to refer to any starch which has been enzymatically hydrolyzed by at least one enzyme capable of cleaving the 1,6-linkages of the starch molecule.

The term waxy or low amylose starch is intended to include a starch containing less than 10% amylose by weight of the dry starch.

The term amylose-containing starch is intended to include any starch containing at least 10% amylose by weight of the dry starch.

The term high amylose is intended to include a starch containing at least 50% amylose by weight of the dry starch.

Gelatinization, as used herein, means a process by which starch is cooked out and loses its granular structure. Granular is intended to mean the structure of native starch in which the starch is not water soluble (still at least partly crystalline) and has birefringence and typically a Maltese cross under polarized light. In high amylose starches, some native granules do not exhibit a Maltese cross, particularly filamentous granules. During gelatinization, as used herein, starch loses its birefringent property as well as any Maltese cross present in its native state.

Crystalline starch is intended to mean a starch that is crystalline, either due to its granular nature or because the gelatinized starch is allowed to recrystallize by methods known in the art.

The present specificationdiscloses methods of treating, preventing or otherwise ameliorating vaginal conditions characterized by, e.g., poor vaginal cell growth, poor vaginal cell differentiation, vaginal atrophy, and low vaginal moisture. The compositions of the invention can be used according to claim 1, e.g., to deliver vaginal moisturizers and in methods for moisturizing the vagina to relieve vaginal dryness and restore health to the vaginal epithelium, and to treat symptoms including vaginal dryness, discomfort, itching, dyspareunia, infection (fungal, bacterial and viral), inflammation, ulcers, discharge, and bleeding.

Treatment of, or treating, a vaginal condition is intended to include the alleviation of or diminishment of at least one symptom typically associated with the vaginal condition. The treatment also includes alleviation or diminishment of more than one symptom. Ideally, the treatment cures, e.g., substantially eliminates one or more symptoms associated with the vaginal condition.

Treatment may also be intended to create a healthy environment in the uterus as, for example, in the vaginal administration of progesterone to aid infertility treatments.

Treatment may also be intended to use the vaginal epithelium as a portal for systemic drug delivery.

The amount administered will vary depending on various factors including, but not limited to, the disease, the weight, the physical condition, the health, the age of the patient, and whether prevention or treatment is to be achieved. Such factors can be readily determined by the clinician employing animal models or other test systems that are available in the art.

Administration of the compositions of the present invention is directly to epithelial cell surfaces of the mucosa. For example, the compositions of the invention can be administered directly to mucosal surfaces. Mucosal surface is vaginal.. In the invention, the epithelial cell surface is vaginal.

Administration of the compositions of the present invention may be in a single dose, in multiple doses, in a continuous or intermittent manner, depending, for example, upon the recipient's physiological condition and is known to skilled practitioners. For prevention of certain conditions or diseases (e.g., vaginal atrophy), administration of the compositions of the invention may be essentially continuous over an indeterminate period of time, for example, at regular intervals for life. Alternatively, the compositions of the invention can be administered continuously for a pre-selected period of time or in a series of spaced doses. Local administration is generally contemplated.

The compositions are prepared by combining the active ingredients in the appropriate concentrations. Other active or inactive agents selected by one of skill in the art can optionally be added. The absolute weight of a given active agent included in a unit dose can vary widely.

Single dosage forms typically are prepared by filling the pellets into hard gelatin or HPMC capsules, by compressing the pellets into tablets or by filling the pellets into an appropriate administration device suitable for vaginal administration.

The compositions of the invention can be administered in the form of an article or carrier such as a vaginal insert, or by way of a syringe-like or plunger-type applicator, tablet, suppository, pessary, powder/talc or other solid, solution, liquid, spray, aerosol, douche, ointment, tampon, foam, cream, gel, paste, microcapsule(s), vaginal sponge, vaginal ring, controlled release formulation, sustained release formulation or bioadhesive gel.

The active ingredients may be used in combination with other therapeutic agents, for example, antifungal agents, antiviral agents, anti-microbial agents, pain relievers, antiinflammatory agents, vitamins (e.g., vitamin B, C or E), aloe vera and the like, whether for conditions described herein or some other condition.

The starches used to prepare the crystalline debranched amylase resistant starch include all starches derived from any native source, any of which may be suitable for use herein. A native starch as used herein, is one as it is found in nature. Also suitable are starches derived from a plant obtained by standard breeding techniques including crossbreeding, translocation, inversion, transformation or any other method of gene or chromosome engineering to include variations thereof. In addition, starch derived from a plant grown from artificial mutations and variations of the above generic composition, which may be produced by known standard methods of mutation breeding, are also suitable herein.

Typical sources for the starches are cereals, tubers, roots, legumes and fruits. The native source can be varieties of corn (maize), pea, potato, sweet potato, banana, barley, wheat, rice, oat, sago, amaranth, tapioca (cassava), arrowroot, canna, and sorghum, as well as low amylose and high amylose varieties thereof. In one embodiment, the high amylose starch contains containing at least 70%, in yet another embodiment at least 80%, and in a further embodiment, at least 90% amylose by weight of the dry starch. In one suitable embodiment, the starch is an amylose-containing starch and in another a high amylose starch with at least 70% amylose by weight.

The starting starch may be dispersed into an aqueous slurry and heated at sufficient temperature and pressure to effect gelatinization. Although gelatinization may be effected by any of the methods known in the art, the preferred method is to force the starch slurry through a jet cooker. Jet-cookers are well known in the industry and consist of a cooking chamber in which the starch slurry is contacted with live steam under elevated temperatures. In one embodiment, gelatinization is complete as determined visually by the total disintegration of granular structure. The gelatinization process disrupts, in whole or in part, the associative bonding of the starch molecules within the raw starch granule. This prepares the starch molecules for debranching by making them more accessible to the debranching enzyme, resulting in more uniformly debranched starch molecules.

After the starch has been gelatinized, it may then be prepared for enzymatic debranching by adjusting the starch solids content to the highest feasible solids level (to keep the amount of water low and to facilitate subsequent drying of the starch). A higher solids starch system may be employed if the starch is processed with adequate mixing to uniformly blend enzyme and starch at the higher solids.

Alternative methods of preparation for enzymatic debranching known in the art may be used. For example, gelatinization is not necessary if using an enzyme which is capable of acting on a granular starch. Another example is using the high solids, single phase process as disclosed in U.S. Patent No. 6,054,302. Yet another alternative example is use of an enzyme immobilized on a solid support.

The temperature and pH of the starch may be adjusted to provide optimum enzyme activity. These parameters will vary depending upon the type and source of enzyme used, the enzyme concentration, the substrate concentration, and the presence or absence of inhibitors.

Any debranching enzymes (or blends of such enzymes) are suitable for use in this application. The enzymes useful in the present invention include without limitation endo-alpha-1,6-glucanohydrolases, such as pullulanase, isoamylase, or any other endo-enzyme that can cleave the 1,6-linkages of the starch molecule. In one embodiment, the enzyme not only cleaves the 1,6 linkages of the starch, but also leaves the 1,4-linkages substantially intact. In one embodiment, the enzyme used is pullulanase. In another embodiment the enzyme used is isoamylase.

The enzymatic hydrolysis of the starch base is carried out using techniques known in the art. The amount of enzyme used is dependent upon the enzyme, i.e., type, source and activity, and base material used as well as the amount of hydrolysis desired. In one embodiment, the enzyme is used in an amount of from about 0.01 to about 1.0%, and in another from about 0.01 to 0.3%, by weight of the starch.

The optimum parameters for enzyme activity will vary depending upon the enzyme used. The rate of enzyme degradation depends upon factors known in the art, including the type of enzyme used, enzyme concentration, substrate concentration, pH, temperature, the presence or absence of inhibitors, and the degree and type of modification. These parameters may be adjusted to optimize the digestion rate of the starch base.

In one embodiment, the enzyme used is pullulanase or pullulan 6-glucanohydrolase, a heat stable enzyme obtained from a species of Bacillus. Pullulanase will catalyze the hydrolysis of the alpha-1,6 linkages in amylopectin, provided that there are at least two glucose units in the side chain. At pH 5.0 the temperature for the aqueous starch dispersion during the enzymatic debranching by the Bacillus pullulanase will be between 25 and 75 °C. If shorter treatment times are desired, the optimum temperature range should be at the upper portion of this range, from 60 to 75 °C (or even higher, if the debranching enzyme is thermally stable at the higher temperatures), or a higher enzyme concentration can be used.

As with other parameters of the enzyme reaction, the preferred and optimum temperature ranges will vary with changes in other parameters that affect enzyme activity, such as substrate concentration and pH, and these can be determined by the practitioner. Buffers, such as acetates, phosphates, citrates, or the salts of other weak acids may be added to ensure that the pH will be at the optimum level throughout the debranching.

Optimum concentrations of enzyme and substrate are governed by the level of enzyme activity, which will vary depending upon the enzyme source, the enzyme supplier, and the concentration of the enzyme provided in commercially available batches.

The enzymatic treatment may be permitted to continue until the desired amount of debranching has occurred and in one embodiment, until essentially complete debranching has occurred; that is, no significant additional debranching will occur using that specific enzyme and other parameters. If desired, the progress of the debranching may be measured by any method known in the art for measuring the degree of enzymatic debranching of starch molecules. The enzyme reaction is continued until the starch is completely debranched. In general, the enzyme reaction will take from about 1 to about 24 hours, particularly about 4 to about 12 hours. The time of the reaction is dependent upon the type of starch used, the amount of enzyme used, and the reaction parameters of solids percent, pH, and temperature.

The amount of hydrolysis may be monitored and defined by measuring the concentration of reducing groups which are freed by alpha-1,6-D-glucanohydrolase activity by methods well known in the art. Other techniques such as monitoring the change in viscosity, iodine reaction, or the change in molecular weight may be used to define the reaction end point. When the starch is completely debranched, the monitored measurement will no longer change. Typically, the starch will be completely debranched when it has been at least about 95%, more particularly at least about 98%, most particularly at least about 99% debranched by weight. The debranched starch will typically have an average chain length of 14-25 glucose units and less than about 0.2%, particularly less than about 0.1% alpha-1,6-D-glucosidic bonds (linkages). Increased debranching will typically lead to a more crystalline starch product.

After the desired amount of starch debranching has been accomplished, the enzyme may be deactivated, for example by pH or heat. Bacillus pullulanase, for example, is rapidly deactivated at temperatures above about 70°C; therefore, the reaction using pullulanase may be conveniently terminated by increasing the temperature of the starch dispersion to at least 75°C for about 15 minutes. Alternatively, the enzyme can be deactivated by adjusting the pH of the starch dispersion to below 3.0 and holding at that pH for about thirty minutes.

After debranching and deactivation of the enzyme, the starch is allowed to crystallize by methods known in the art, such as by retrogradation. This may be done by any method known in the art, and is conventionally done by allowing the starch to stand, and in one embodiment is done by allowing the starch to stand at temperatures below room temperature, such as at refrigerator temperatures.

The starch may be recovered using methods known in the art, particularly by extrusion, filtration, centrifugation, or drying, including spray drying, freeze drying, flash drying or air drying, more particularly by filtration or flash drying and in one particular embodiment is by extrusion or flash drying. The drying may be done to a partial extent, for example, to 60%-80% solids, and the resultant product then dried further. Alternatively, the starch can be dried to 100% solids, conventionally 10-15% moisture by weight of the dry starch. It is important to control the crystallization, typically by controlling retrogradation and drying, in order to obtain the necessary degree of crystallinity which is important to the present invention. It is further important that the method of drying and other post-crystallization processes do not substantially destroy the crystals.

The particle size of the dried powder may be adjusted using methods known in the art including, without limitation, by agglomeration. The particle size of the dried powder may be controlled during manufacture by methods known in the art to obtain an average (mean) particle size of, in one embodiment, at least about 25 microns, and no more than about 90 microns.

Optionally, the moisture content may be adjusted to allow for improved flow and compaction. Crystallization may be controlled using methods known in the art, such as by controlling retrogradation and drying, in order to obtain the desired degree of crystallinity. The starch must be at least partially crystalline in order to work in a spheronization process.

In another embodiment, the starch product is isolated by adding an inorganic salt to the starch dispersion and incubating the mixture at 50 to 100°C. The salt can be any known salt that will not interfere with starch retrogradation and that will act to help draw out the water of gelatinization, permitting the association of the linear starch molecules. Suitable salts include without limitation sodium sulfate, ammonium sulfate or magnesium sulfate, and sodium chloride. In one embodiment, the salts are added to the deactivated starch slurry in a minimum of 10% of the solids content.

The starches may also be converted and include without limitation fluidity or thin-boiling starches prepared by oxidation, acid hydrolysis, and enzyme hydrolysis. These processes are well known in the art and may be accomplished either before or after debranching.

The starch may also be further modified, either before or after the enzymatic hydrolysis. Such modification may be physical, enzyme, or chemical modification. Physical modification includes by shearing or thermally inhibiting, for example by the process described in U.S. Patent No. 5,725,676.

Chemical modification includes without limitation, crosslinking, acetylation and organic esterification, hydroxyalkylation, phosphorylation and inorganic esterification, cationic, anionic, nonionic, and zwitterionic modifications, and succination. Such modifications are known in the art, for example in Modified Starches: Properties and Uses, Ed. Wurzburg, CRC Press, Inc., Florida (1986).

Any starch base having suitable properties for use herein may be purified by any method known in the art to remove starch off flavors and colors that are native to the polysaccharide or created during processing. Suitable purification processes for treating starches are disclosed in the art and include without limitation alkali washing techniques. Such purification methods are also useful on the debranched starch.

If purification of the debranched starch composition is desired, reaction impurities and by-products may be removed by dialysis, filtration, centrifugation or any other method known in the art for isolating and concentrating starch compositions. For example, the degraded starch may be washed using techniques known in the art to remove soluble low molecular weight fractions, such as oligosaccharides, resulting in more highly crystalline starch. In one embodiment, a narrow range of molecular weights is isolated and allowed to crystallize, to result in a more crystalline starch.

The resultant solution is typically adjusted to the desired pH according to its intended end use. In general, the pH is adjusted to from about 5.0 to about 7.5, and in one embodiment from about 6.0 to about 7.0, using techniques known in the art. Further, any short chain amylose which precipitated out of the starch dispersion may be redispersed or removed.

The crystalline debranched starch described herein is uniquely functional as a pharmaceutical excipient for use in extrusion-spheronization, which process will typically comprise the following steps:
a) the mixing of one or more debranched starches, and optionally other excipients and/or one or more active agents, to obtain a uniform mixture in form of dry powder to which a suitable amount of liquid is added to obtain a moistened plastically deformable mass;
b) the extrusion of the mixture obtained from the step a) through a perforated mesh in order to obtain cylindrical extrudates having desired length and diameter;
c) the spheronization of the extrudates in order to obtain a product in the form of spherical pellets;
d) the drying of the pellets;
e) the optional depositing of at least one active agent on the surface of the pellets; and
f) the optional coating of the pellets.

The starch may be used as the sole excipient or in combination with microcrystalline cellulose and/or other cellulose derivatives. In one embodiment, the debranched starch is used in an amount of from 10 to 100%, in another embodiment from 25 to 95%, and in a third embodiment from 60 to 90%, by weight of the plastically deformable mass on a dry basis. In another embodiment, the debranched starch is used as the primary excipient (greater than 50% of the excipients by weight) and is used in combination with a binder, the binder being present in an amount of up to 8% by weight of the plastically deformable mass on a dry basis.

In one embodiment, binders are added to the formulation in an amount of up to about 25%, and in another embodiment in an amount of up to about 15%, and in yet another embodiment in an amount of up to about 8%, all by weight of the plastically deformable mass on a dry weight basis to help the material withstand the frictional forces of spheronization and result in a larger pellet size. In one embodiment, the binder is present in an amount of from 4-8% by weight of the plastically deformable mass on a dry weight. Binders include those commonly known in the art and in one embodiment is selected from the group consisting of hydroxypropyl methylcellulose, drum dried waxy corn starch, hydroxypropyl cellulose and polyvinylpyrrolidone and in another from hydroxypropyl methyl cellulose and drum dried waxy corn starch.

Optionally, a plasticizer may be added to improve the surface properties of the pellets and in one embodiment is added in an amount of up to 30%, in one embodiment in an amount of up to 25%, and in another up to 15%, by weight plastically deformable mass on a dry basis. In one aspect of the invention, the plasticizer is a polyol and in another is sorbitol.

The liquid added to form a moistened mass for extrusion may include any liquid substance or mix (solution or emulsion) of liquids of normal pharmaceutical use able to moisten the powder mix, as for example water, aqueous solutions having different pH, organic solvents of normal pharmaceutical use (for example, alcohols, chlorinated solvents, and oils). In one embodiment, the liquid is water. The wet mass may be prepared using any equipment known in the art including without limitation a planetary mixer, high-shear mixer, sigma blade mixer, or continuous granulator.

The liquid is added in any desirable amount, typically in an amount of from 20 to 55%, and more typically from about 30 to 45% based on the wet weight of the plastically deformable mass.

In one aspect of the invention, the active agent and/or excipients may be dissolved, dispersed and/or emulsified in such liquids.

The moistened mass is extruded through a perforated mesh in order to produce extrudates (cylindrical filaments). The port of the meshes determines the diameter of the extrudates and in one embodiment is from about 0.2 mm to 3 mm and in another from about 0.5 mm to about 2 mm. The extrusion may be carried out using single screw, double screw, "sieve and basket" kind, "roll extruder", "ram extruder" extruders or any other pharmaceutically acceptable means to produce extrudates. In one embodiment of this invention a double screw coaxial extruder may be used.

In one aspect of the invention, the extruded mass may be re-extruded prior to spheronization in order to obtain a higher densification level of the extrudates.

The extrudates obtained by extrusion are then spheronized. The spheronization device consists of a hollow cylinder with a horizontal rotating plate. The extrudates are broken in short segments which are transformed to pellets on the upper surface of a rotating plate, and in one aspect of the invention at a velocity ranging from about 200 rpm to about 2,000 rpm. The pellets may be dried in any pharmaceutically acceptable way, such as drying at room temperature and may be accomplished in any apparatus known in the art including without limitation, in an oven, a fluidized bed, or a microwave oven.

Use of debranched starches provides a yield of greater than 80% and in one embodiment greater than 90%. Yield is intended to mean the percent of the extruded composition which resulted in usable pellets, that is pellets which are substantially spherical solid particles whose diameter size range from about 100 microns to about 3 mm. In one embodiment, the diameter of the pellets ranges from about 0.70 to 1.40 mm. Fines and pellets which are not of the desired size may be removed from the resultant pellets by techniques known in the art, such as sieving.

Pellets will typically have an aspect ratio of between about 1 and 1.3 and have a pellet friability of less than 2%, more typically less that 0.5%. Pellet aspect ratio means the ratio of the largest and the smallest diameter of a pellet. Friability means the tendency of the pellets to flake off during handling resulting in the formation of dust.

The pellets may be used as they are or may be coated. In one embodiment, the coating is with an active agent, either the same as or different from the active agent, if any, within the pellet. In another embodiment, the pellets are coated for functional purposes, which include without limitation to obtain a controlled release effect, to improve the shelf-life, and for identification purposes. The optional coating may contain an active agent as well as at least one functional ingredient.

The active agents may be distributed inside the pellets and/or they may be deposited on the surface of the pellets by techniques normally used in the pharmaceutical arts, including without limitation by spray drying and coating.

In the case that an active is distributed inside the microparticles, it will typically range from about 0.1% to about 95% by weight of the microparticles.

Additional pharmaceutical excipients known in the art may be added to the pharmaceutical dosage form to impart satisfactory processing, disintegration, or other characteristics to the formulation. Such excipients include, but are not limited to, flow enhancers, surfactants, lubricants and glidants, disintegrants, and colorants. These excipients are well known in the art and are limited only by compatibility and characteristics desired.

Lubricants and glidants include talc, magnesium stearate, calcium stearate, stearic acid, glyceryl behenate, glycerol monostearate, mineral oil, polyethylene glycol, sodium stearyl fumarate, stearic acid, vegetable oil, zinc stearate, and silicon dioxide.

Disintegrants suitable for the present invention include starches, algins, gums, croscarmelose, crospovidone, sodium starch glycolate, sodium lauryl sulfate, polacrilin potassium, and methylcellulose.

If the final desired product is other than a pharmaceutical dosage form, alternative additives known to those arts may be present.

It is to be understood that other methods of granulation known in the art which involve high shear may be used to form the pellets including fluid-bed and rotogranulation, centrifugal granulator, or high-shear granulation.

### EXAMPLES

The following examples are presented to further illustrate and explain the present invention and should not be taken as limiting in any regard. All percents used are on a weight/weight basis.

### Example 1

Samples A, B, and C, containing 50% metronidazole, 25 % miconazole nitrate, and 20 % medroxyprogesteronacetate, respectively, and having the composition set forth in Table 1, were prepared. The compositions also contained water, in amounts added as required.

**Table 1**

| Ingredient | Sample A (% w/w) | Sample B (% w/w) | Sample C (% w/w) |
|---|---|---|---|
| Active: | | | |
| Metronidazole | 50 | | |
| Miconazole nitrate | | 25 | |
| Medroxyprogesteronacetate | | | 20 |
| Hydroxypropylmethylcellulose | 7 | 4.5 | 6 |
| (HPMC) | | | |
| (Methocel E15L/EP Pharm, Colorcon) | | | |
| Sorbitol | 4.3 | 11.25 | 10.5 |
| Modified starch | | | |
| (VELOX™ MCS, Henkel Corp.) | 38.7 | 59.25 | 63.5 |

Pellets were prepared by mixing the drug, HPMC, sorbitol and the modified starch. Next the powder mixture was granulated for 10 min at 60 rpm by means of a planetary mixer with a K-shaped mixing arm using demineralized water as the granulating liquid. The water level was determined based on preliminary tests and corresponds to the level resulting in the highest yield. Water is added during the first 30 s of the wet massing phase. To ensure uniform water distribution during wet massing, the material adhering to the mixing bowl was regularly removed. The wet mass was extruded at an extrusion speed of 50 rpm using a single screw extruder (Dome extruder lab model DEL1, Fuji Paudal, Tokyo, Japan), equipped with a dome-shaped extrusion screen (thickness: 1.2 mm, perforation diameter: 1 mm). The extrudates were spheronized for 3 min in a spheronizer having a friction plate with cross-hatched geometry (Caleva Model 15, Caleva, Sturminster Newton, Dorset, UK). Pellets were dried for 20 min at 60 °C in a fluid-bed drier (Uniglatt, Glatt, Binzen, Germany).

After drying the pellets were prepared as a single dosage form by filling the pellets into hard gelatin capsules, by filling the pellets into HPMC capsules, by compressing the pellets into tablets or by filling the pellets into an appropriate administration device for suitable for vaginal administration.

### Example 2

In this example, non-disintegrating microcrystalline cellulose pellets (MCC) and disintegrating starch based pellets were analysed for their in-vivo behavior (distribution and retention in the vagina). Pellets were also compared with a powder formulation.

### Materials:

Microcrystalline cellulose pellets (MCC) (Cellets^{®} 500 µm, Pharmatrans-Sanaq, Basel, Switzerland) were used as non-disintegrating pellets.

A high amylose, crystalline and resistant starch (VELOX™ MCS, Henkel Corporation, New Jersey, USA) was used as the main excipient (84.9 %) of the starch-based pellets.

Hydroxypropylmethylcellulose (HPMC) (Methocel^{®} E15 LV EP Pharm, Colorcon, Dartford, UK) (4.9 %) was used as a binder and sorbitol (Sorbidex^{®} P 16616, Cerestar, Vilvoorde, Belgium) (10.2 %) was added to modify the consistency of the wet mass (A. Dukié, R. Mens, P. Adriaensens, P. Foreman, J. Gelan, J.P. Remon, C. Vervaet, Eur. J. Pharm. Biopharm. 66(1) (2007) 83-94).

Demineralized water was used as a granulation liquid.

Riboflavine sodium phosphate (Certa, Braine l'Alleud, Belgium) was used as marker at a concentration of 5% for easy visualization and quantification.

### Preparation of Starch-based Pellets:

Starch-based pellets were produced by extrusion/ spheronization. Dry mixing was performed in a Turbula^{®} mixer (model T2A, W.A. Bachofen, Basel, Switzerland) for 15 min. The powder mixture was granulated with demineralized water for 10 min using a planetary mixer (Kenwood Chef, Hampshire, UK) with a K-shaped mixing arm. Water was added during the first 30 s of the wet massing phase. To ensure uniform water distribution, the material adhering to the mixing bowl was regularly removed. The wet mass was extruded at an extrusion speed of 50 rpm using a single screw extruder (Dome extruder lab model DG-L1, Fuji Paudal, Tokyo, Japan) equipped with a dome-shaped extrusion screen with 0.4 mm perforations. The extrudates were spheronized in a spheronizer having a friction plate with cross-hatched geometry (Caleva Model 15, Caleva, Sturminster Newton, Dorset, UK). Spheronization time was 3 min and spheronization speed 1000 rpm. The pellets were overnight dried in an oven (Memmert, Schwabach, Germany) at 40°C. The fraction of 315 - 800 µm was separated using a sieve shaker (Retsch, Haan, Germany).

### Preparation of powder formulation:

10 % lactose (α-Pharma, Braine-l'Alleud, Belgium)/ 20 % skim milk (Difco, Becton Dickinson, MA, USA) was dispersed in demineralized water, poured in petri dishes and freeze dried using an Amsco Finn Aqua GT4 freeze dryer. The dispersion was frozen to -45°C within 175 min at 1000 mbar. The primary drying was performed at -15°C and at a pressure varying between 0.8 and 1 mbar during 13h, followed by the secondary drying at elevated temperature (10°C) and reduced pressure (0.1-0.2 mbar) for 7h.

Clinical trials were performed with healthy volunteers. Volunteering women were screened to exclude gynaecological and systemic pathology, including cervico-vaginal infections or microflora alterations according to a standardized protocol. Study participants adhered to a strict protocol that involved an extensive list of behaviors they were to refrain from to avoid interference with the vaginal formulation, including abstinence from coitus for 48 hours prior to administration and 24h after administration of the product, no use of vaginal hygiene products (spray, foams,...) and no depilation of pubic hair. To avoid interference with menstrual or withdrawal bleeding, study participants adhered to a continuous oral contraceptive regimen during the conduct of the studies.

Two formulations of pellets and one powder formulation were compared: non-disintegrating MCC pellets, fast disintegrating starch pellets and a freeze-dried powder. These were loaded into dissolvable capsules and administered high in the vagina at the fornix. All volunteers lay down for 3h or 6h following administration and underwent colposcopy after 3h or 6h, and 24h.

In vivo behaviour of the pellets was assessed through colposcopy with photographs taken that covered the entire ectocervical and vaginal mucosa. During colposcopy the vagina was swabbed at three areas: at the fornix, the mid portion of the vault mucosa and the introitus, to evaluate spreading of the vaginal formulation and percent of riboflavin sodium phosphate (RSP) retrieved by swabbing at the fornix, mid vagina and introitus was determined. In some cases the capsules, although adhering well to the vaginal mucosa and weakened by absorption of vaginal fluid, had not yet released their contents by the time of the initial colposcopy. At this time release was accomplished by gently touching the weakened capsule with a speculum.

Results showed that the non disintegrating MCC pellets clustered together around the fornix after 3 h and no spreading over the lateral walls to the introitus occurred. After 24 h a limited number of pellets (<50) were retrieved mostly located at the midportion of the vagina and around the introitus. Volunteers reported most release of pellets between 5-7 h after administration, mostly during toilet visit and showering. Chatterton et al. also reported most loss of a vaginal cream formulation during urination (B.E. Chatterton, S. Penglis, J.C. Kovacs, B. Presnell, B. Hunt, Int. J. Pharm. 271(1-2) (2004) 137-143). Hence, despite the small particle size and the spherical form of the pellets, no good spreading and retention of the pellets was obtained with the MCC pellets.

Starch based pellets also clustered around the fornix 6 h after administration, but the start of disintegration and spreading of the pellets was observed. After 24 h, for almost all volunteers a complete coverage of the vaginal mucosa with disintegrating pellets was observed. Analysis of the swabs showed that after 6h, RSP was almost only detected around the fornix, but after 24 h RSP was retrieved at the fornix, mid vagina and introitus, though still the highest concentration around the fornix (Table 2 and 3).

The powder formulation was mostly spread around the fornix after 6 h, but some distribution to the middle anterior and posterior wall of the mid vagina was observed (Table 4 and 5). No coverage of the side walls was seen. Colposcopy after 24 h showed very little RSP marked powder. Once the powder was dispersed it behaved like a liquid and was cleared rapidly from the vagina.

**Table 2**

| % RSP retrieved at 6 hours | | | | |
|---|---|---|---|---|
| Pellets | Fornix | Mid vagina | Introitus | Total |
| Volunteer 1 | 0.14 | 0.01 | 0.00 | 0.15 |
| Volunteer 2 | 0.00 | 0.00 | 0.00 | 0.00 |
| Volunteer 3 | 0.60 | 0.00 | 0.00 | 0.60 |
| Volunteer 4 | 0.00 | 0.00 | 0.00 | 0.00 |
| Volunteer 5 | 0.48 | 0.02 | 0.00 | 0.49 |
| Mean | 0.24 | 0.01 | 0.00 | 0.25 |

**Table 3**

| % RSP retrieved at 24 hours | | | | |
|---|---|---|---|---|
| Pellets | Fornix | Mid vagina | Introitus | Total |
| Volunteer 1 | 0.70 | 0.25 | 0.04 | 0.99 |
| Volunteer 2 | 0.49 | 0.10 | 0.10 | 0.69 |
| Volunteer 3 | 0.15 | 0.02 | 0.00 | 0.17 |
| Volunteer 4 | 0.14 | 0.01 | 0.00 | 0.15 |
| Volunteer 5 | 0.34 | 0.13 | 0.02 | 0.49 |
| Mean | 0.37 | 0.10 | 0.03 | 0.50 |

**Table 4**

| % RSP retrieved at 6 hours | | | | |
|---|---|---|---|---|
| Powder | Fornix | Mid vagina | Introitus | Total |
| Volunteer 1 | 0.30 | 0.05 | 0.00 | 0.35 |
| Volunteer 2 | 0.04 | 0.00 | 0.00 | 0.04 |
| Volunteer 3 | 0.47 | 0.15 | 0.17 | 0.79 |
| Volunteer 4 | 0.01 | 0.04 | 0.02 | 0.07 |
| Volunteer 5 | 0.82 | 0.00 | 0.00 | 0.82 |
| Mean | 0.33 | 0.05 | 0.04 | 0.41 |

**Table 5**

| % RSP retrieved at 24 hours | | | | |
|---|---|---|---|---|
| Powder | Fornix | Mid vagina | Introitus | Total |
| Volunteer 1 | 0.07 | 0.01 | 0.00 | 0.08 |
| Volunteer 2 | 0.00 | 0.00 | 0.00 | 0.00 |
| Volunteer 3 | 0.05 | 0.02 | 0.00 | 0.07 |
| Volunteer 4 | 0.03 | 0.03 | 0.00 | 0.06 |
| Volunteer 5 | 0.29 | 0.08 | 0.00 | 0.37 |
| Mean | 0.09 | 0.03 | 0.00 | 0.12 |

## Claims

1. A composition comprising pellets for use in the treatment of a disease by vaginal administration, said pellets comprising a debranched starch, which has been enzymatically hydrolyzed by at least one enzyme capable of cleaving the 1,6-linkages of the starch molecule, and a therapeutic agent.

2. The composition of claim 1 in the form of a pessary, a tablet or a capsule.

3. The composition of claim 1 wherein the pellets are prepared by extrusion/spheronization.

4. The composition of claim 1 wherein the therapeutic agent is a pharmacologically active agent.

5. The composition of claim 3 wherein the therapeutic agent is mixed in with the debranched starch during the preparation of the pellet.

6. The composition of claim 1 wherein the therapeutic agent is deposited on the surface of the pellet.

7. The composition of claim 1 wherein the debranched starch is prepared using a high amylose starch.

8. The composition of claim 1 wherein the debranched starch is prepared using pullulanase

9. A medicament comprising the composition of any one of claims 1 to 8 for use in the treatment of a disease by vaginal administration.

10. A medicament comprising the composition of any of claims 1 to 8 for use in the treatment of a disease according to claim 9, wherein the medicament is for the treatment of bacterial and fungal infections.

11. A medicament comprising the composition of any of claims 1 to 8 for use in the treatment of a disease according to claim 9, wherein the medicament is for the treatment of vaginosis and vaginitis.

12. A medicament comprising the composition of any of claims 1 to 8 for use in the treatment of a disease according to claim 9, wherein the medicament is for the treatment of low vaginal moisture, vaginal dryness, vaginal acidity or vaginal atrophy.

## Patentansprüche

1. Zusammensetzung, umfassend Pellets zur Verwendung beim Behandeln einer Krankheit durch vaginale Gabe, wobei die Pellets eine entzweigte Stärke, die durch wenigstens ein Enzym, das in der Lage ist, die 1,6-Verbindungen des Stärkemoleküls zu spalten, enzymatisch hydrolysiert worden ist, und ein Therapeutikum umfassen.

2. Zusammensetzung nach Anspruch 1 in der Form eines Pessars, einer Tablette oder einer Kapsel.

3. Zusammensetzung nach Anspruch 1, wobei die Pellets durch Extrudieren/Sphäronisieren hergestellt werden.

4. Zusammensetzung nach Anspruch 1, wobei das Therapeutikum ein pharmakologisch wirksames Mittel ist.

5. Zusammensetzung nach Anspruch 3, wobei das Therapeutikum beim Herstellen des Pellets mit der entzweigten Stärke vermischt wird.

6. Zusammensetzung nach Anspruch 1, wobei das Therapeutikum auf der Oberfläche des Pellets abgelagert ist.

7. Zusammensetzung nach Anspruch 1, wobei die entzweigte Stärke unter Verwendung einer hoch amylosehaltigen Stärke hergestellt wird.

8. Zusammensetzung nach Anspruch 1, wobei die entzweigte Stärke unter Verwendung einer Pullulanase hergestellt wird.

9. Medikament, umfassend die Zusammensetzung nach einem der Ansprüche 1 bis 8 zur Verwendung beim Behandeln einer Krankheit durch vaginale Gabe.

10. Medikament, umfassend die Zusammensetzung nach einem der Ansprüche 1 bis 8 zur Verwendung beim Behandeln einer Krankheit nach Anspruch 9, wobei das Medikament zum Behandeln von bakteriellen und Pilzinfektionen vorgesehen ist.

11. Medikament, umfassend die Zusammensetzung nach einem der Ansprüche 1 bis 8 zur Verwendung beim Behandeln einer Krankheit nach Anspruch 9, wobei das Medikament zum Behandeln von Vaginose und Vaginitis vorgesehen ist.

12. Medikament, umfassend die Zusammensetzung nach einem der Ansprüche 1 bis 8 zur Verwendung beim Behandeln einer Krankheit nach Anspruch 9, wobei das Medikament zum Behandeln von geringer Scheidenfeuchtigkeit, Scheidentrockenheit, vaginaler Azidität oder vaginaler Atrophie vorgesehen ist.

## Revendications

1. Composition contenant des comprimés destinés à être utilisés dans le traitement d'une maladie par administration vaginale, lesdits comprimés contenant un amidon déramifié qui a été hydrolysé enzymatiquement par au moins une enzyme capable de cliver les liaisons 1-6 de la molécule d'amidon, et un agent thérapeutique.

2. Composition selon la revendication 1 sous forme d'un pessaire, d'un comprimé ou d'une capsule.

3. Composition selon la revendication 1 dans laquelle les comprimés sont préparés par extrusion/sphéronisation.

4. Composition selon la revendication 1 dans laquelle l'agent thérapeutique est un agent pharmacologiquement actif.

5. Composition selon la revendication 3 dans laquelle l'agent thérapeutique est mélangé dans l'amidon déramifié pendant la préparation du comprimé.

6. Composition selon la revendication 1 dans laquelle l'agent thérapeutique est déposé sur la surface du comprimé.

7. Composition selon la revendication 1 dans laquelle l'amidon déramifié est préparé en utilisant un amidon à haute teneur en amylose.

8. Composition selon la revendication 1 dans laquelle l'amidon déramifié est préparé en utilisant de la pullulanase.

9. Médicament contenant la composition selon l'une quelconque des revendications 1 à 8, destiné à être utilisé dans le traitement d'une maladie par administration vaginale.

10. Médicament contenant la composition selon l'une quelconque des revendications 1 à 8, destiné à être utilisé dans le traitement d'une maladie selon la revendication 9, le médicament étant destiné au traitement d'infections bactériennes et fongiques.

11. Médicament contenant la composition selon l'une quelconque des revendications 1 à 8, destiné à être utilisé dans le traitement d'une maladie selon la revendication 9, le médicament étant destiné au traitement de la vaginose et de la vaginite.

12. Médicament contenant la composition selon l'une quelconque des revendications 1 à 8, destiné à être utilisé dans le traitement d'une maladie selon la revendication 9, le médicament étant destiné au traitement de la faible humidité vaginale, de la sécheresse vaginale, de l'acidité vaginale ou de l'atrophie vaginale.
